# EUROPEAN PATENT APPLICATION

(11) **EP 4 751 697 A1**
(43) Date of publication of application: **03.06.2026**
(21) Application number: 24859571.2
(22) Date of filing: 21.08.2024
(51) Int. Cl.: A61J 1/20, A61M 5/162

(54) **VIAL ADAPTER**

(30) Priority: 25.08.2023 JP 2023137609
(71) Applicant: Terumo Kabushiki Kaisha, Tokyo 151-0072 (JP)
(72) Inventor: KAWANA, Hiroe, Nakakoma-gun, Yamanashi 409-3853 (JP); KURIYAMA, Tasuku, Nakakoma-gun, Yamanashi 409-3853 (JP)
(74) Representative: KIPA AB
(86) International application number: PCT/JP2024/029646
(87) International publication number: WO 2025/047538

(57) **Abstract**

A vial adapter according to the present disclosure includes a spike that is elongated from a proximal end to a distal end to define a longitudinal direction and that has a liquid flow path inside. The liquid flow path includes a discharge port opened to an outer peripheral surface of the spike in a cross-section orthogonal to the longitudinal direction. The discharge port includes an enlarged opening in which an opening angle defined by two straight lines connecting a central axis of the spike and two ends of the discharge port in a circumferential direction around the central axis gradually increases toward the distal end in the cross-section.

## Description

### Technical Field

The present disclosure relates to a vial adapter.

### Background Art

There is known a vial adapter having a spike that is elongated from a proximal end to a distal end to define a longitudinal direction and that has a liquid flow path therein, the liquid flow path having a discharge port opened to an outer peripheral surface of the spike in a cross-section perpendicular to the longitudinal direction (see, for example, Patent Literature 1).

### Citation List

### Patent Literature

Patent Literature 1: JP 2010-124898 A

### Summary of Invention

### Technical Problem

According to the vial adapter described in Patent Literature 1, the spike can be inserted into a rubber stopper provided at the mouth of a vial, and a liquid can be injected into the vial through the discharge port. However, in the vial adapter described in Patent Literature 1, the discharge port may be closed by the inner surface of a fixing portion of the rubber stopper fitted to the mouth of the vial, and the discharge of the liquid through the discharge port may be hindered. In addition, since the discharge port is provided on the outer peripheral surface of the spike in the cross-section orthogonal to the longitudinal direction of the spike, the edge of the discharge port is caught, and it may be difficult to insert the spike through the rubber stopper.

An object of the present disclosure is to provide a vial adapter having a spike in which discharge of a liquid through a discharge port is less likely to be interfered with and which is easily inserted into a rubber stopper.

### Solution to Problem

A vial adapter according to a first aspect of the present disclosure is
(1)
   a vial adapter including
   a spike that is elongated from a proximal end to a distal end to define a longitudinal direction and that has a liquid flow path inside, in which
   the liquid flow path includes a discharge port opened to an outer peripheral surface of the spike in a cross-section orthogonal to the longitudinal direction, and
   the discharge port includes an enlarged opening in which an opening angle defined by two straight lines connecting a central axis of the spike and two ends of the discharge port in a circumferential direction around the central axis gradually increases toward the distal end in the cross-section.

A vial adapter according to one embodiment of the present disclosure is
(2)
the vial adapter according to (1), in which the spike includes a baffle surface that guides, toward the discharge port, a liquid flowing through the liquid flow path from the proximal end toward the distal end.

A vial adapter according to one embodiment of the present disclosure is
(3)
the vial adapter according to (1) or (2), in which the discharge port has a proximal end opening in which the opening angle is constant regardless of a position in the longitudinal direction, the proximal end opening being continuous with the enlarged opening on a side of the proximal end.

A vial adapter according to one embodiment of the present disclosure is
(4)
   the vial adapter according to any one of (1) to (3), in which
the spike includes:
   a sharp portion including a tapered distal end portion; and
   a discharge port forming portion positioned closer to the proximal end with respect to the sharp portion and formed with the discharge port, and
   the discharge port forming portion has an outer peripheral surface having a shape along an ellipse in the cross-section, the ellipse having a major axis with a length that is constant regardless of a position in the longitudinal direction.

A vial adapter according to one embodiment of the present disclosure is
(5)
the vial adapter according to (4), in which in the discharge port forming portion, a length of a minor axis of the ellipse decreases toward the distal end.

A vial adapter according to one embodiment of the present disclosure is
(6)
   the vial adapter according to (4) or (5), in which the spike has a base portion continuous with the discharge port forming portion on a side of the proximal end, and
the base portion has a minimum thickness that is equal to or larger than a maximum thickness of the discharge port forming portion.

A vial adapter according to one embodiment of the present disclosure is
(7)
the vial adapter according to (6), in which the base portion has a circular outer peripheral surface in the cross-section.

A vial adapter according to one embodiment of the present disclosure is
(8)
the vial adapter according to any one of (1) to (7), in which the spike includes a gas flow path having an opening end on an outer surface of the spike.

A vial adapter according to one embodiment of the present disclosure is
(9)
the vial adapter according to (8), in which the opening end of the gas flow path is positioned closer to the distal end with respect to the discharge port.

### Advantageous Effects of Invention

The present disclosure can provide a vial adapter having a spike in which discharge of a liquid through a discharge port is less likely to be interfered with and which is easily inserted into a rubber stopper.

### Brief Description of Drawings

Fig. 1 is an external view illustrating a vial adapter according to an embodiment of the present disclosure.
Fig. 2 is an enlarged external view illustrating a spike of the vial adapter illustrated in Fig. 1.
Fig. 3 is an external view of the spike illustrated in Fig. 2 as viewed from a viewpoint different from that in Fig. 2.
Fig. 4 is a cross-sectional view taken along line a-a in Fig. 3.
Fig. 5A is a cross-sectional view taken along line b-b in Fig. 4.
Fig. 5B is a cross-sectional view taken along line c-c in Fig. 4.
Fig. 5C is a cross-sectional view taken along line d-d in Fig. 4.
Fig. 5D is a cross-sectional view taken along line e-e in Fig. 4.
Fig. 5E is a cross-sectional view taken along line f-f in Fig. 4.
Fig. 5F is a cross-sectional view taken along line g-g in Fig. 4.
Fig. 6 is an external view illustrating a state where the vial adapter illustrated in Fig. 1 is connected to a vial.
Fig. 7 is an external view, partially in cross-section, illustrating a part of Fig. 6 in cross-section.
Fig. 8 is an enlarged view of a portion Z in Fig. 7.

### Description of Embodiments

Hereinafter, an embodiment of a vial adapter according to the present disclosure will be described by way of example with reference to the drawings. In the drawings, the same components are denoted by the same reference signs.

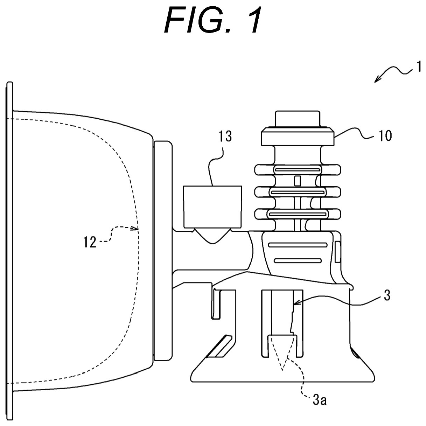

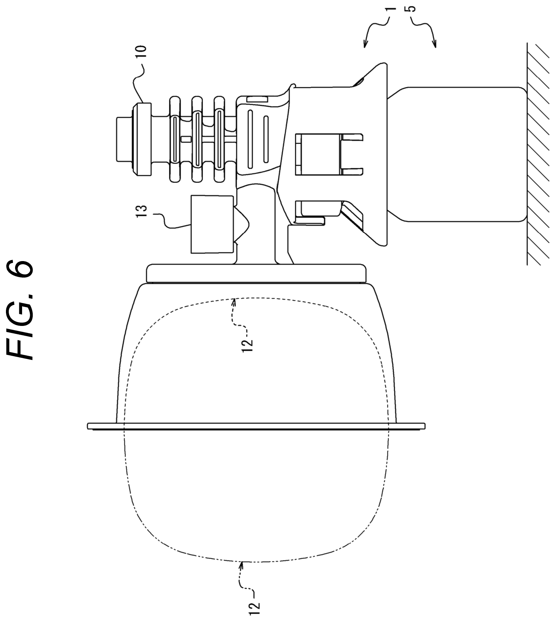

Fig. 1 is an external view illustrating a vial adapter 1 as one embodiment of the vial adapter according to the present disclosure. Fig. 6 is an external view illustrating a state where the vial adapter 1 illustrated in Fig. 1 is connected to a vial 5. As illustrated in Fig. 6, the vial adapter 1 is used by being connected to the vial 5. The vial adapter 1 is used when, for example, a liquid is supplied into the vial 5. The vial adapter 1 is also used when, for example, a liquid is sucked from the interior of the vial 5.

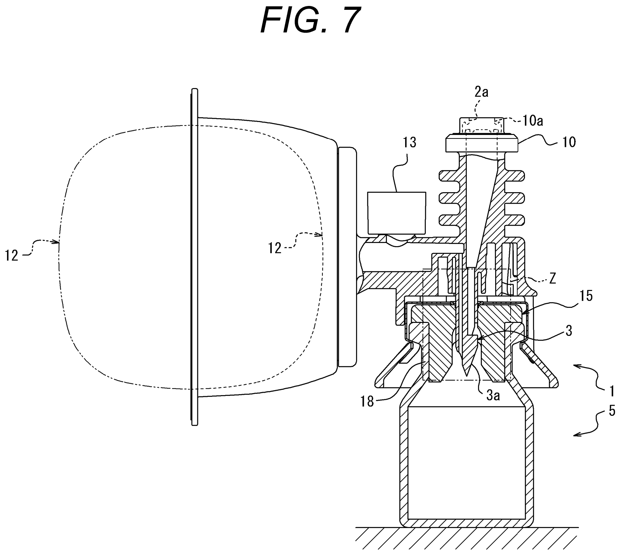

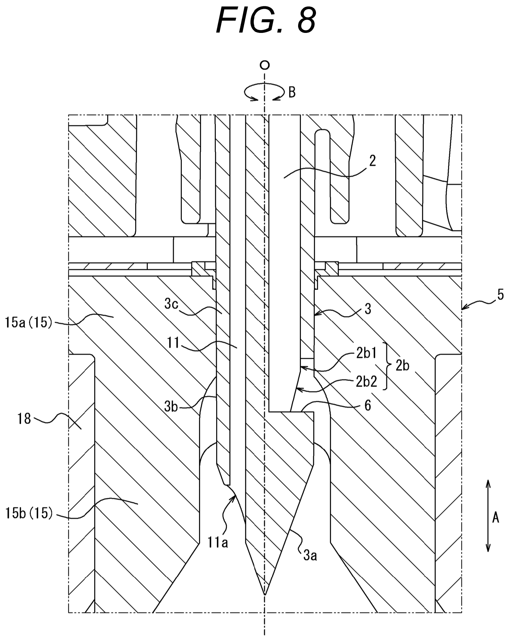

Fig. 7 is a cross-sectional external view illustrating a part of the vial adapter 1 and the vial 5 illustrated in Fig. 6 in cross-section. Fig. 8 is an enlarged view of a portion Z in Fig. 7. As illustrated in Figs. 7 and 8, a rubber stopper 15 is attached to a mouth 18 of the vial 5 according to the present embodiment. The vial 5 according to the present embodiment is sealed by the rubber stopper 15 attached to the mouth 18. The rubber stopper 15 in the present embodiment includes a cover portion 15a having a substantially circular flat shape and disposed so as to cover the mouth 18, and a fixing portion 15b protruding from the cover portion 15a and fitted to the inner peripheral surface of the mouth 18. The spike 3 is inserted into the cover portion 15a of the rubber stopper 15, by which the vial adapter 1 according to the present embodiment is connected to the vial 5.

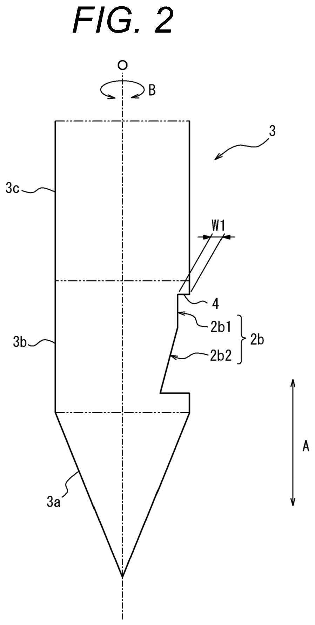

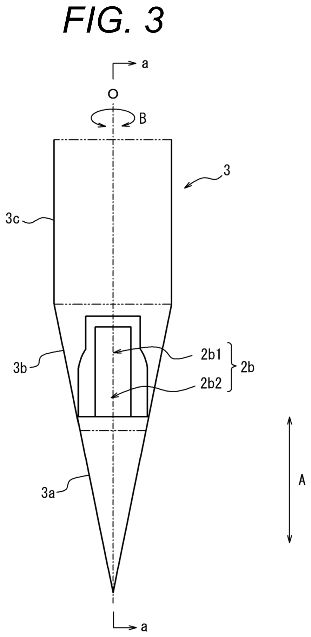

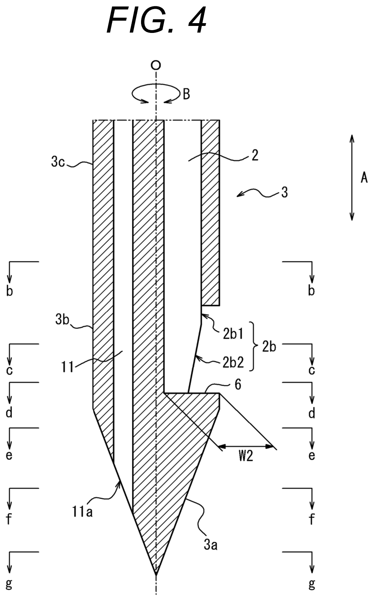

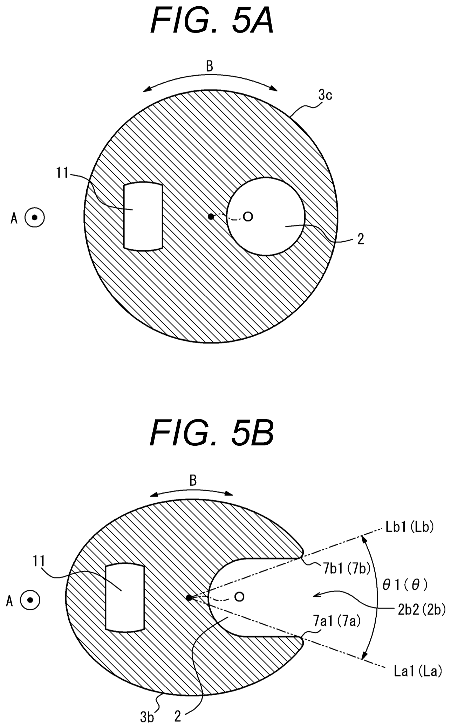

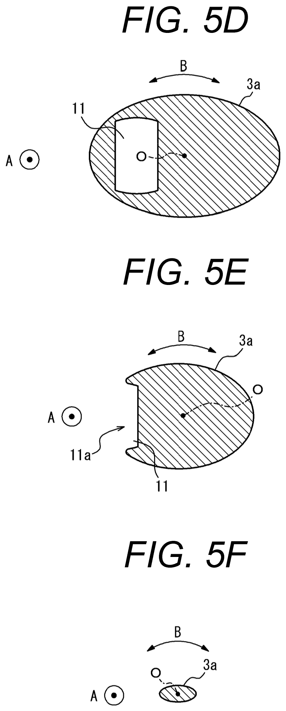

Fig. 2 is an enlarged external view illustrating the spike 3 of the vial adapter 1 illustrated in Fig. 1. Fig. 3 is an external view of the spike 3 illustrated in Fig. 2 as viewed from a viewpoint different from that in Fig. 2. Fig. 4 is a cross-sectional view taken along line a-a in Fig. 3. Figs. 5A to 5F are cross-sectional views taken along lines b-b to g-g in Fig. 4.

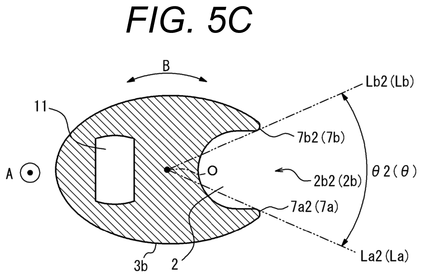

As illustrated in Figs. 2 to 4, the vial adapter 1 has the spike 3 that is elongated from a proximal end to a distal end to define a longitudinal direction A and that has a liquid flow path 2 therein. As illustrated in Figs. 2 to 4, the spike 3 has a sharp portion 3a including a tapered distal end portion. As illustrated in Figs. 2 to 4, the liquid flow path 2 has a discharge port 2b that opens to the outer peripheral surface of the spike 3 in a cross-section orthogonal to the longitudinal direction A (hereinafter, sometimes simply referred to as a "cross-section"). In the cross-section, the discharge port 2b has an enlarged opening 2b2 in which an opening angle θ (see Figs. 5B and 5C) defined by two straight lines La and Lb connecting a central axis O of the spike 3 and two ends 7a and 7b of the discharge port 2b along a circumferential direction B around the central axis O gradually increases toward the distal end side of the spike 3 (hereinafter, sometimes simply referred to as "distal end side").

For example, in the cross-sectional view illustrated in Fig. 5B, an opening angle θ1 is defined by two straight lines La1 and Lb1 connecting the central axis O of the spike 3 and two ends 7a1 and 7b1 of the discharge port 2b along the circumferential direction B around the central axis O. In the cross-sectional view illustrated in Fig. 5C, an opening angle θ2 is defined by two straight lines La2 and Lb2 connecting the central axis O of the spike 3 and two ends 7a2 and 7b2 of the discharge port 2b along the circumferential direction B around the central axis O. The opening angle θ2 is larger than the opening angle θ1.

The discharge port 2b includes the enlarged opening 2b2, and thus, even when the fixing portion 15b of the rubber stopper 15 approaches or comes into contact with the discharge port 2b of the spike 3, a gap is easily formed between the enlarged opening 2b2 of the discharge port 2b and the fixing portion 15b of the rubber stopper 15 in a direction orthogonal to the longitudinal direction A as illustrated in Fig. 8, for example, whereby the discharge port 2b is hardly closed. As a result, the discharge of the liquid through the discharge port 2b is less likely to be interfered with. The same effect can be obtained when, for example, the inner peripheral surface of the mouth 18 of the vial 5 instead of the fixing portion 15b of the rubber stopper 15 approaches or comes into contact with the discharge port 2b. In addition, since the opening angle θ increases toward the distal end side (in other words, the opening angle θ decreases toward the proximal end side of the spike 3 (hereinafter, sometimes simply referred to as "proximal end side"), it is easy to reduce a width W1 (see Fig. 2) of a notch surface 4 defining the end on the proximal end side of the discharge port 2b. Since the width W1 of the notch surface 4 is reduced, the notch surface 4 is less likely to be caught by the rubber stopper 15 when the spike 3 is inserted into the rubber stopper 15, whereby the spike 3 can be easily inserted into the rubber stopper 15. Furthermore, since the opening angle θ gradually increases toward the distal end side, a level difference is not formed between the two ends 7a and 7b of the enlarged opening 2b of the discharge port 2b along the circumferential direction B around the central axis O. Therefore, when the spike 3 is inserted into the rubber stopper 15, the two ends 7a and 7b of the enlarged opening 2b of the discharge port 2b along the circumferential direction B around the central axis O are less likely to be caught by the rubber stopper 15, whereby the spike 3 can be easily inserted into the rubber stopper 15.

As illustrated in Fig. 4, the spike 3 includes a baffle surface 6 that guides a liquid flowing through the liquid flow path 2 from the proximal end side toward the distal end side in the longitudinal direction A toward the discharge port 2b. More specifically, the baffle surface 6 in the present embodiment is a surface that faces the proximal end side of the spike 3 for defining an end of the liquid flow path 2 on the distal end side. According to the above configuration, the liquid flowing in the liquid flow path 2 from the proximal end side toward the distal end side in the longitudinal direction A is brought into contact with the baffle surface 6, so that the liquid can be guided in the direction intersecting the longitudinal direction A, that is, in the direction in which the discharge port 2 is located. Therefore, the liquid can be easily discharged from the discharge port 2b. As a result, when being supplied to the vial 5, the liquid can flow along the side wall of the vial 5, which can prevent bubbling.

Although the baffle surface 6 in the present embodiment is a flat surface orthogonal to the longitudinal direction A, the baffle surface 6 may not be orthogonal to the longitudinal direction A as long as the baffle surface 6 faces the proximal end side. That is, the baffle surface 6 may be an inclined surface that inclines toward the distal end of the spike 3 as it extends from the central axis O to the discharge port 2b side or an inclined surface that inclines toward the proximal end of the spike 3 as it extends from the central axis O to the discharge port 2b side in a longitudinal section including the central axis O of the spike 3, passing through the discharge port 2b, and parallel to the longitudinal direction A. However, the configuration in which the baffle surface 6 is a flat surface orthogonal to the longitudinal direction A as in the present embodiment can prevent the liquid from dripping not along the side wall of the vial 5 as compared with an inclined surface that inclines toward the distal end of the spike 3 as it extends from the central axis O to the discharge port 2b side. In addition, this configuration can also prevent the liquid from staying in the liquid flow path 2 as compared with the inclined surface that inclines toward the proximal end of the spike 3 as it extends from the central axis O to the discharge port 2b side.

As illustrated in Figs. 2 to 4, the discharge port 2b in the present embodiment has a proximal end opening 2b1 which is continuous with the enlarged opening 2b2 on the proximal end side and in which the size of the opening angle θ described above is constant regardless of the position in the longitudinal direction A. According to the above configuration, the length of each of the two ends 7a and 7b of the discharge port 2b along the circumferential direction B around the central axis O can be easily reduced as compared with the case where the discharge port 2b is entirely constituted by the enlarged opening 2b2. Since the length of each of the two ends 7a and 7b of the discharge port 2b is reduced along the circumferential direction B around the central axis O, the sliding length of the spike 3 and the rubber stopper 15 when the spike 3 is inserted into the rubber stopper 15 can be reduced, whereby the spike 3 can be easily inserted into the rubber stopper 15.

As illustrated in Figs. 2 to 4, the spike 3 in the present embodiment has a discharge port forming portion 3b which is positioned proximal to the sharp portion 3a and in which the discharge port 2b is formed. As illustrated in Figs. 5B and 5C, the outer peripheral surface of the discharge port forming portion 3b in the present embodiment has a shape along an ellipse in the cross-section, and the length of the major axis of the ellipse is constant regardless of the position in the longitudinal direction A. As illustrated in Figs. 5B and 5C, the discharge port 2b in the present embodiment is opened in the length direction of the major axis of the ellipse. In the present disclosure, the "shape along an ellipse" includes an ellipse and a shape that will become an ellipse by adding an imaginary line for supplementing a missing part.

According to the above configuration, since the outer peripheral surface of the discharge port forming portion 3b has a shape along an ellipse in the cross-section, the area of the cross-section of the spike 3 can be easily reduced, and the spike 3 can be easily inserted into the rubber stopper 15, as compared with the case where, for example, the outer peripheral surface of the discharge port forming portion 3b has a shape along a circle in the cross-section. In addition, the length of the major axis of the ellipse described above is constant regardless of the position in the longitudinal direction A, and this configuration makes it easy to ensure the arrangement space for the liquid flow path 2 and a gas flow path 11 to be described later inside the spike 3. More specifically, as illustrated in Figs. 5B and 5C, by arranging the liquid flow path 2 and the gas flow path 11 side by side on the major axis of the above-described ellipse, it is easy to ensure the arrangement space for the liquid flow path 2 and the gas flow path 11 inside the spike 3. Further, in a case where the discharge port 2b is opened in the length direction of the major axis of the above-described ellipse as in the present embodiment (see Figs. 5B and 5C), the length of the major axis of the ellipse is constant regardless of the position in the longitudinal direction A, so that a width W2 (see Fig. 4) of the baffle surface 6 can be easily ensured as compared with the case where the length of the major axis of the ellipse decreases toward the distal end side.

In the discharge port forming portion 3b in the present embodiment, the length of the minor axis of the above-described ellipse decreases toward the distal end side (see Figs. 5B and 5C). According to the above configuration, when the liquid flow path 2 and the gas flow path 11 are arranged side by side on the major axis of the ellipse as in the present embodiment, it is possible to efficiently reduce the area of the cross-section of the spike 3 while ensuring the arrangement space for the liquid flow path 2 and the gas flow path 11 inside the spike 3, whereby the spike 3 can be easily inserted into the rubber stopper 15.

As illustrated in Figs. 2 to 4, the spike 3 has a base portion 3c continuous with the discharge port forming portion 3b on the proximal end side. The minimum thickness of the base portion 3c in the present embodiment is equal to or larger than the maximum thickness of the discharge port forming portion 3b. Here, the thickness of the spike 3 means the maximum width of the spike 3 in the cross-section. As will be described later, an outer peripheral surface of the base portion 3c in the present embodiment is circular in the cross-section. Therefore, the thickness of the base portion 3c in the present embodiment is equal to the diameter of the circular shape. The outer peripheral surface of the discharge port forming portion 3b in the present embodiment has a shape along an ellipse. Therefore, the thickness of the discharge port forming portion 3b in the present embodiment is equal to the length of the major axis of the ellipse. The minimum thickness of the base portion 3c means the minimum value of the thickness of the base portion 3c. The thickness of the base portion 3c in the present embodiment is constant in the longitudinal direction A. Therefore, the minimum thickness of the base portion 3c in the present embodiment is the thickness of the base portion 3c at any position in the longitudinal direction A. Furthermore, the maximum thickness of the discharge port forming portion 3b means the maximum value of the thickness of the discharge port forming portion 3b. The thickness of the discharge port forming portion 3b in the present embodiment is constant in the longitudinal direction A. Therefore, the maximum thickness of the discharge port forming portion 3b in the present embodiment is the thickness of the discharge port forming portion 3b at any position in the longitudinal direction A. Due to the configuration in which the minimum thickness of the base portion 3c is equal to or larger than the maximum thickness of the discharge port forming portion 3b as described above, the rubber stopper 15 and the outer peripheral surface of the base portion 3c of the spike 3 can be brought into close contact with each other without a gap in a state where the spike 3 is inserted into the rubber stopper 15. Therefore, in a case where, for example, the vial 5 is turned upside down and a liquid is sucked from the vial 5 to a suction/discharge device through the liquid flow path 2 of the vial adapter 1, a leakage of the liquid from between the rubber stopper 15 and the spike 3 can be prevented.

As illustrated in Fig. 5A, the outer peripheral surface of the base portion 3c in the present embodiment is circular in the cross-section. According to the above configuration, the rubber stopper 15 and the outer peripheral surface of the base portion 3c of the spike 3 inserted into the rubber stopper 15 can be more reliably brought into close contact with each other without a gap as compared with the case where the outer peripheral surface of the base portion 3c is not circular in the cross-section, and thus, a leakage of liquid from the gap between the rubber stopper 15 and the spike 3 can be further prevented.

In the present embodiment, the sharp portion 3a has an outer peripheral surface having a shape along an ellipse in the cross-section and is configured such that the lengths of the major axis and the minor axis of the ellipse decrease toward the distal end side (see Figs. 5D to 5F). According to the above configuration, the distal end portion of the spike 3 can be formed to have a tapered shape, so that the spike 3 can be easily inserted into the rubber stopper 15.

In the present embodiment, the sharp portion 3a, the discharge port forming portion 3b, and the base portion 3c are defined by dividing the spike 3 in the longitudinal direction A.

As illustrated in Fig. 7, the vial adapter 1 according to the present embodiment has a medical device connection portion 10 formed with a connection port 2a communicating with the liquid flow path 2. According to the above configuration, in a state where the medical device connection portion 10 is connected to a suction/discharge device such as a syringe and the spike 3 is connected to the vial 5, a liquid can be supplied from the suction/discharge device to the vial 5 through the liquid flow path 2 of the vial 5 and mixed with the content in the vial 5 to form a mixed liquid. The vial 5 can then be turned upside down to suction the liquid from the vial 5 through the liquid flow path 2 of the vial adapter 1 to the suction/discharge device.

As illustrated in Fig. 4, the spike 3 in the present embodiment has a gas flow path 11 having an opening end 11a on the outer surface of the spike 3. According to the above configuration, when the spike 3 is connected to the vial 5 and a liquid is supplied to the vial 5 through the liquid flow path 2, gas can be discharged from the vial 5 through the gas flow path 11. In addition, gas can be introduced into the vial 5 through the gas flow path 11 when the vial 5 is turned upside down to suction the liquid from the vial 5 through the liquid flow path 2 of the vial adapter 1 to the suction/discharge device.

The opening end 11a of the gas flow path 11 in the present embodiment is positioned on the opposite side of the discharge port 2b with respect to the center of the spike 3 as viewed in the longitudinal direction A. This configuration can prevent the liquid discharged to the vial 5 through the liquid flow path 2 from entering the gas flow path 11 when the liquid is supplied to the vial 5 through the liquid flow path 2 in a state where the spike 3 is connected to the vial 5. Further, with this configuration, when the vial 5 is turned upside down to suction the liquid from the vial 5 to the suction/discharge device through the liquid flow path 2 of the vial adapter 1 and to introduce a gas from the gas flow path 11 to the vial 5, bubbling of the liquid with the gas can be suppressed.

As illustrated in Fig. 4, the opening end 11a of the gas flow path 11 in the present embodiment is positioned distal to the discharge port 2b. With this configuration, when the vial 5 is turned upside down to suction the liquid from the vial 5 to the suction/discharge device through the liquid flow path 2 of the vial adapter 1 and to introduce a gas from the gas flow path 11 to the vial 5, bubbling of the liquid with the gas can be suppressed.

The medical device connection portion 10 in the present embodiment is of a closed type in which a valve body 10a is disposed at the connection port 2a. With the above configuration, it is possible to prevent the entry of foreign matters into the liquid flow path 2 when nothing is connected to the medical device connection portion 10.

The vial adapter 1 according to the present embodiment has a balloon 12 that has an internal space communicating with the gas flow path 11 and that is selectively expandable and contractible. With the above configuration, by supplying the liquid to the vial 5 through the liquid flow path 2, the gas discharged from the vial 5 through the gas flow path 11 is temporarily held in the internal space of the balloon 12, and then, can be returned from the internal space of the balloon 12 to the vial 5 through the gas flow path 11 by sucking the liquid from the vial 5 through the liquid flow path 2. Therefore, when, for example, an anticancer agent or the like is handled in the vial 5, a harmful gas discharged from the vial 5 can be prevented from being discharged to the external space.

The vial adapter 1 according to the present embodiment includes a suction valve 13 that allows inflow of gas from the external space to the gas flow path 11 and prevents outflow of the gas from the gas flow path 11 to the external space. With such a configuration, even in a case where a gas to be introduced into the vial 5 is not sufficient in the internal space of the balloon 12 due to the influence of temperature change or the like when a liquid is sucked from the vial 5 through the liquid flow path 2, a gas can be introduced from the suction valve 13 to make up for the gas shortage.

The present disclosure is not limited to the above-described embodiment and may be modified in various manners without departing from the gist of the present disclosure.

Therefore, the vial adapter 1 according to the embodiment can be variously modified as long as it is a vial adapter 1 including a spike 3 that is elongated from the proximal end to the distal end to define a longitudinal direction A and that has a liquid flow path 2 inside, the liquid flow path 2 including a discharge port 2b opened to an outer peripheral surface of the spike 3 in a cross-section orthogonal to the longitudinal direction A, the discharge port 2b including an enlarged opening 2b2 in which an opening angle θ defined by two straight lines La and Lb connecting a central axis O of the spike 3 and two ends 7a and 7b of the discharge port 2b in a circumferential direction B around the central axis O gradually increases toward the distal end in the cross-section orthogonal to the longitudinal direction A.

### Industrial Applicability

The present disclosure relates to a vial adapter.

### Reference Signs List

- 1: Vial adapter
- 2: Liquid flow path
- 2a: Connection port
- 2b: Discharge port
- 2b1: Proximal end opening
- 2b2: Enlarged opening
- 3: Spike
- 3a: Sharp portion
- 3b: Discharge port forming portion
- 3c: Base portion
- 4: Notch surface
- 5: Vial
- 6: Baffle surface
- 7a: End
- 7b: End
- 10: Medical device connection portion
- 10a: Valve body
- 11: Gas flow path
- 11a: Opening end
- 12: Balloon
- 13: Suction valve
- 15: Rubber stopper
- 15a: Cover portion
- 15b: Fixing portion
- 18: Mouth
- A: Longitudinal direction of spike
- B: Circumferential direction around central axis of liquid flow path
- La: Straight line
- Lb: Straight line
- O: Central axis of liquid flow path
- W1: Width of notch surface
- W2: Width of baffle surface

## Claims

1. A vial adapter comprising a spike that is elongated from a proximal end to a distal end to define a longitudinal direction and that has a liquid flow path inside, wherein
the liquid flow path includes a discharge port opened to an outer peripheral surface of the spike in a cross-section orthogonal to the longitudinal direction, and
the discharge port includes an enlarged opening in which an opening angle defined by two straight lines connecting a central axis of the spike and two ends of the discharge port in a circumferential direction around the central axis gradually increases toward the distal end in the cross-section.

2. The vial adapter according to claim 1, wherein the spike includes a baffle surface that guides, toward the discharge port, a liquid flowing through the liquid flow path from the proximal end toward the distal end.

3. The vial adapter according to claim 1 or 2, wherein the discharge port has a proximal end opening in which the opening angle is constant regardless of a position in the longitudinal direction, the proximal end opening being continuous with the enlarged opening on a side of the proximal end.

4. The vial adapter according to claim 1 or 2, wherein the spike includes
a sharp portion including a tapered distal end portion, and
a discharge port forming portion positioned closer to the proximal end with respect to the sharp portion and formed with the discharge port, and
the discharge port forming portion has an outer peripheral surface having a shape along an ellipse in the cross-section, the ellipse having a major axis with a length that is constant regardless of a position in the longitudinal direction.

5. The vial adapter according to claim 4, wherein in the discharge port forming portion, a length of a minor axis of the ellipse decreases toward the distal end.

6. The vial adapter according to claim 4, wherein the spike has a base portion continuous with the discharge port forming portion on a side of the proximal end, and
the base portion has a minimum thickness that is equal to or larger than a maximum thickness of the discharge port forming portion.

7. The vial adapter according to claim 6, wherein the base portion has a circular outer peripheral surface in the cross-section.

8. The vial adapter according to claim 1 or 2, wherein the spike includes a gas flow path having an opening end on an outer surface of the spike.

9. The vial adapter according to claim 8, wherein the opening end of the gas flow path is positioned closer to the distal end with respect to the discharge port.
